# EUROPEAN PATENT SPECIFICATION

(11) **EP 3 308 821 B1**
(45) Date of publication and mention of the grant of the patent: **29.01.2020**
(21) Application number: 17195480.3
(22) Date of filing: 09.10.2017
(51) Int. Cl.: A61M 21/00

(54) **A SUPPORT FOR HOSTING OF A TREATED SUBJECT, PARTICULARLY A CHAISE LONGUE**
UNTERLAGE ZUM HOSTEN EINES BEHANDELTEN PATIENTEN, INSBESONDERE ZUSAMMENKLAPPBARE LIEGE
SUPPORT POUR L'ACCUEIL D'UN SUJET TRAITÉ, NOTAMMENT UNE CHAISE LONGUE

(30) Priority: 12.10.2016 IT 201600102496
(43) Date of publication of application: 18.04.2018
(73) Proprietor: Innersee S.r.l., 10129 Torino (IT)
(72) Inventor: BIANCOFIORE, Roberto, 10129 Torino (IT)
(74) Representative: De Bonis, Paolo

(56) References cited:
- AT-A1- 508 627
- US-A- 5 387 178

## Description

### Field of the invention

The present invention relates to a support for for hosting of a treated subject, in particular a so-called chaise longue, which is configured for administration of treatments in wellness and fitness centres, gymns, medical structures, and the like.

In particular, the invention has been developed with reference to supports that include one or more sensory-stimulation units via which the treatment is administered to a user or a patient. Prior art supports are known, e.g., from AT 508 627 A1 and US 5,387,178.

### Prior art and general technical problem

Supports of the type referred to above that use stimulation of the senses (hearing, sight, touch, smell) of the user or of the patient in general comprise sensory-stimulation units, the position and type of which depend upon the sensory apparatus that is to be stimulated. For instance, a unit for stimulating hearing is in general set so as to be in the proximity and/or oriented towards the ears of the user, which can be assumed as occupying a preset position according to the conformation of the support (for example, a position corresponding to the headrest).

Furthermore, according to the sensory apparatus that is to be stimulated, there may be used stimulation devices that operate on completely different bases. In particular, it is possible to have stimulation devices operation of which is independent of consumable materials, and devices operation of which depends upon consumable materials.

Constituting an example of the first type is an acoustic diffuser or speaker that is supplied with the electric-power mains, whereas an example of the second type may correspond to a device for stimulation of smell that includes cartridges or phials that release essences into the air (possibly combined with a forced-air-circulation device).

This mainly poses two kinds of problems, namely
- a problem of configuration, in so far as - except possibly for touch stimulation - stimulation of the remaining sensory receptors of the human body basically imposes location of the stimulation units in the area of the head of the user, with the consequent need to provide all the equipment necessary for stimulation in that area; this leads to a high concentration of devices in a single area, which renders the supplies of consumable material inaccessible and renders replacement thereof impracticable, if not by stopping - or even dismantling - some parts of the treatment support; and
- a problem of continuity of the treatment provided, this being at least in part a consequence of the first problem, in so far as stimulation devices that depend upon the level of the supplies of consumable material for their operation may occasionally find themselves in conditions of depleted supply during administration of the treatment itself; on account of the problem of configuration referred to above, this entails the need to interrupt temporarily the treatment, ask the user or patient to get off the support (with consequent probable loss of all the benefits achieved in the meantime), and replace the consumable materials; only subsequent to this can the treatment resume, with evident detriment to the user (or at least dissatisfaction of the user).

### Object of the invention

The object of the present invention is to solve the technical problems mentioned previously.

In particular, the object of the present invention is to provide a support for hosting a treated subject in which it is possible to reintegrate the supplies of consumable materials necessary for administration of the treatment, without this having an impact on administration of the treatment itself.

### Summary of the invention

The object of the present invention is achieved by a support for hosting a treated subject having the features that form the subject of one or more of the ensuing claims, which form an integral part of the technical disclosure provided herein in relation to the invention.

In particular, the object of the invention is achieved by a support for hosting of a treated subject according to claim 1.

### Brief description of the drawings

The invention will now be described with reference to the annexed drawings, which are provided purely by way of non-limiting example and in which:
- Figure 1 is a perspective view of a treatment support according to a preferred embodiment of the invention;
- Figure 2 is a perspective view according to the arrow II of Figure 1;
- Figure 2A is a view corresponding to that of Figure 2, but illustrating some components in see-through view;
- Figure 3 is a longitudinal sectional view of the treatment support according to the invention;
- Figure 4 is a perspective view of an ensemble indicated by the arrow IV in Figure 2;
- Figure 5 is an exploded perspective view corresponding to the representation of Figure 4;
- Figure 6 is a perspective view of an ensemble indicated by the arrow VI in Figure 1;
- Figure 7 is a perspective view according to the arrow VII of Figure 6; and

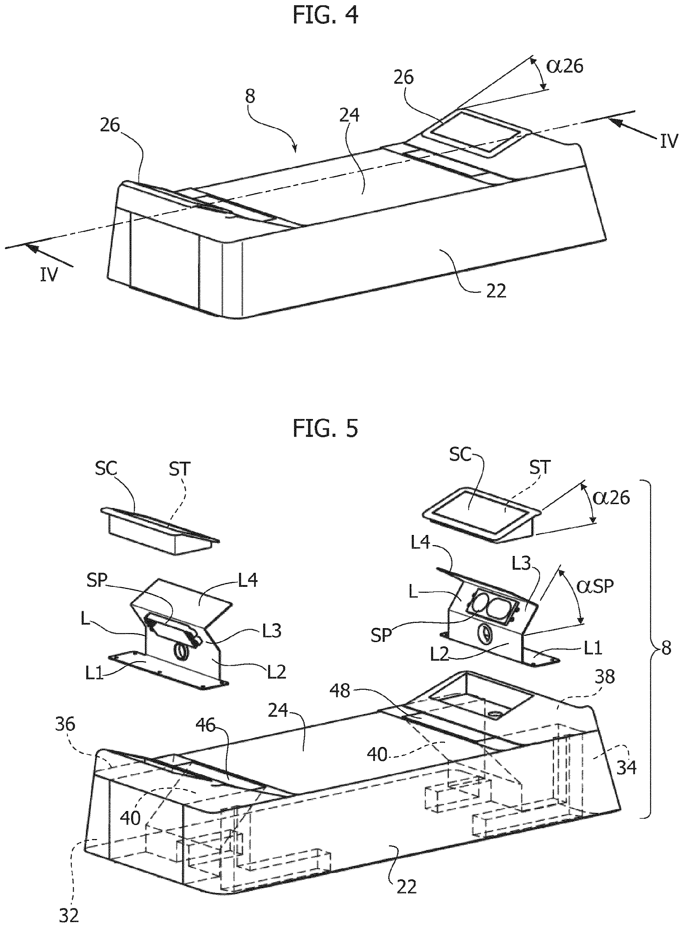

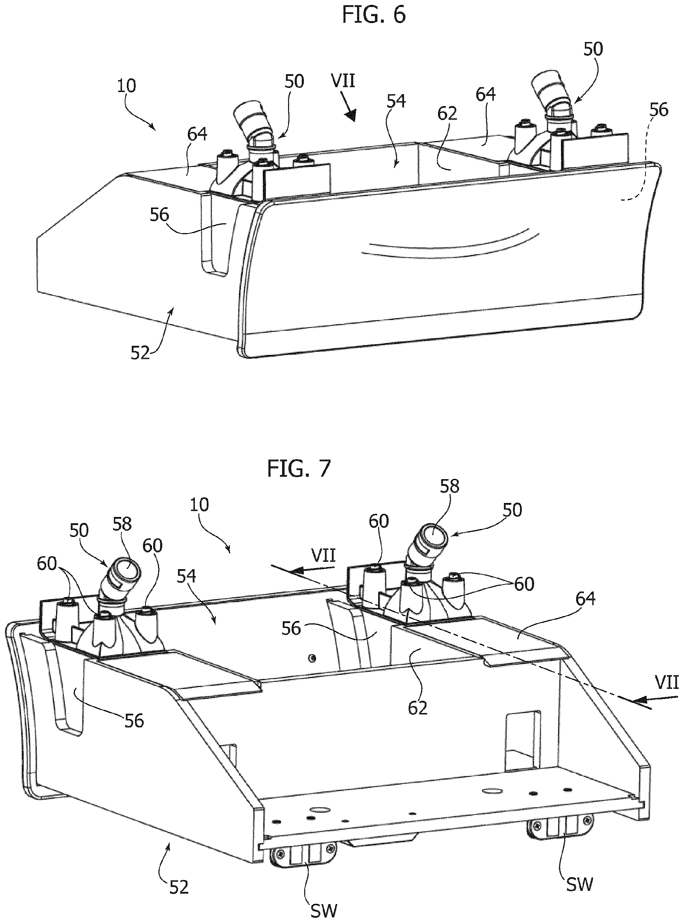

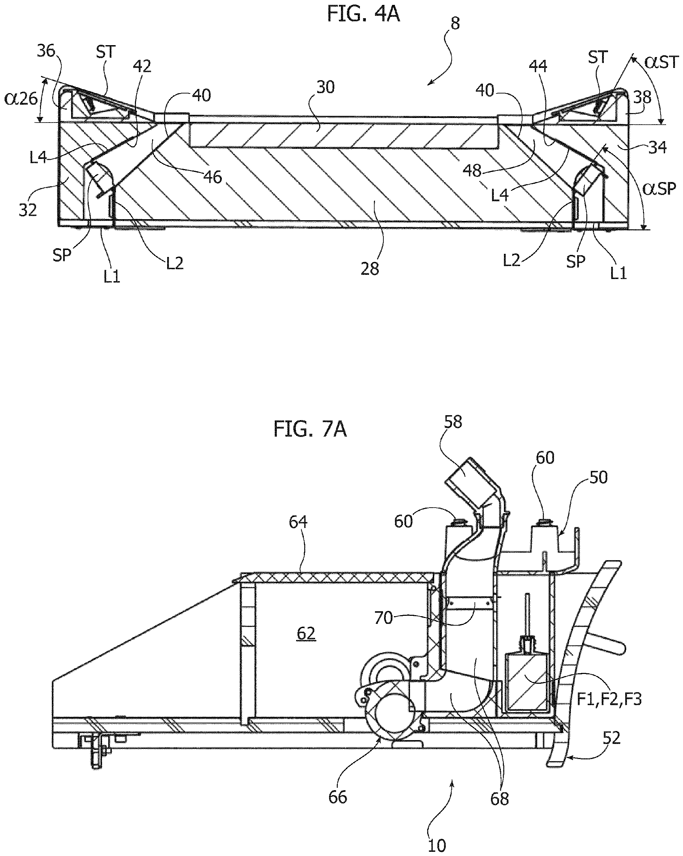

- Figures 4A and 7A are cross-sectional views according to the traces IV-IV and VII-VII of Figures 4 and 7, respectively.

### Detailed description of preferred embodiments

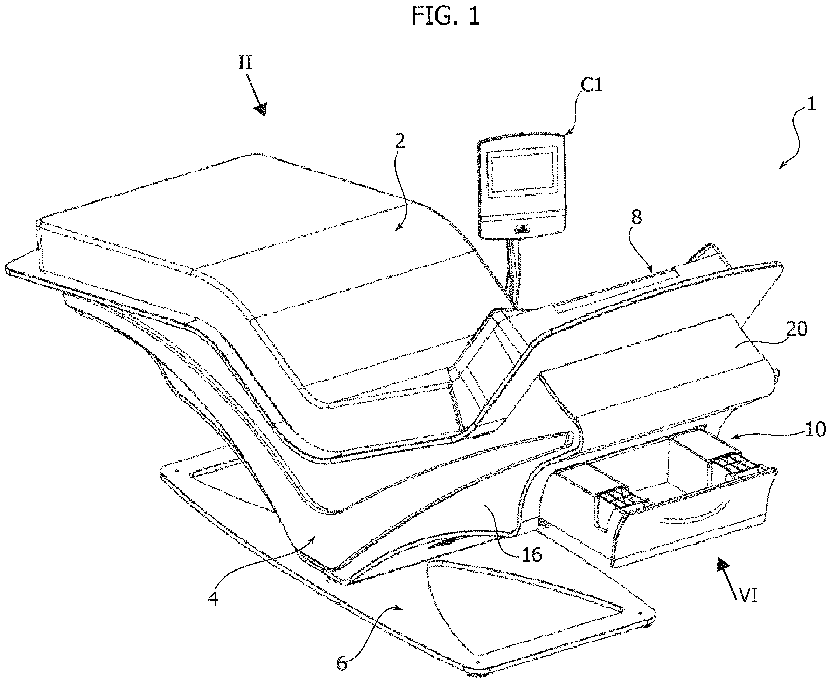

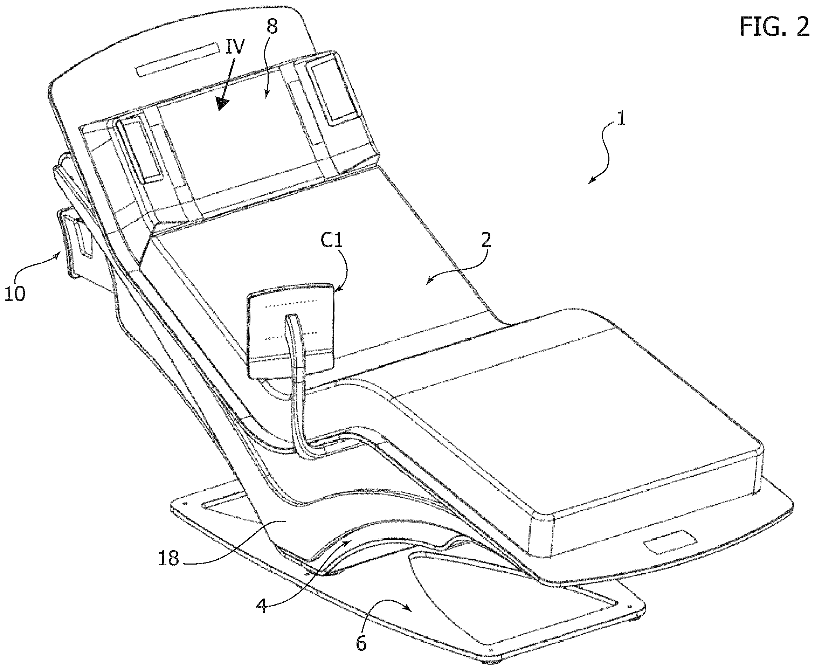

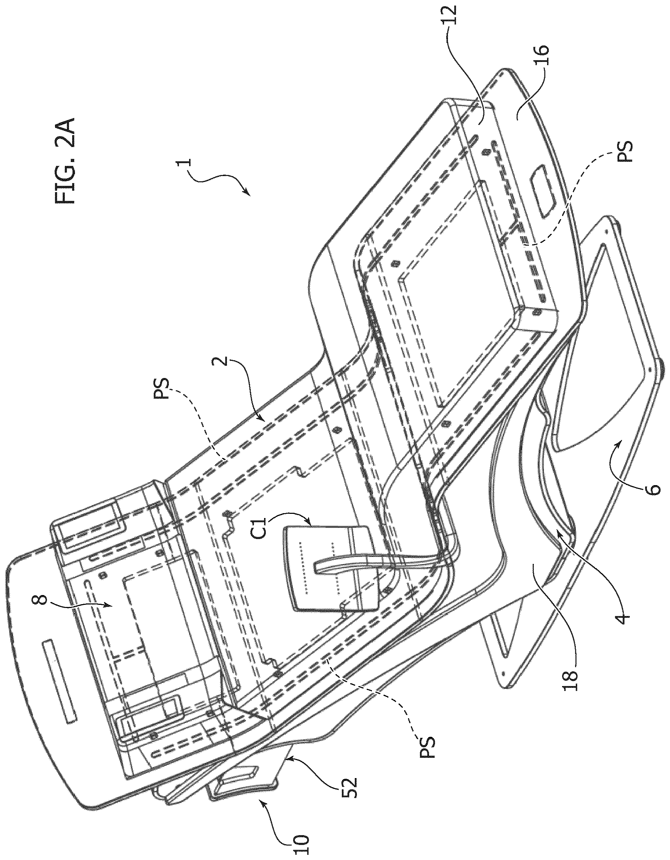

With reference to Figures 1 and 2, a support for hosting a treated subject according to the invention is designated as a whole by the reference number 1. In this preferred embodiment, the support 1 is configured as a chaise longue, but it remains understood that it may be made as a treatment couch or as a chair with a more upright seat.

The support 1 includes a hosting seat 2 that is configured for accommodating a user to whom the treatment is to be administered, a supporting frame 4, and a base 6 that supports the frame 4 and the hosting seat 2 (as well as all the additional structures) at a certain height from the floor.

The treatment support 1 further includes a first stimulation unit designated by the reference number 8 and a second stimulation unit designated by the reference number 10. The first stimulation unit 8 is set at one end of the hosting seat 2, in particular at an end where the head of a user, who is accommodated on the support and to whom the treatment is to be administered, rests. In this way, the first stimulation unit 8 itself provides a headrest for the user.

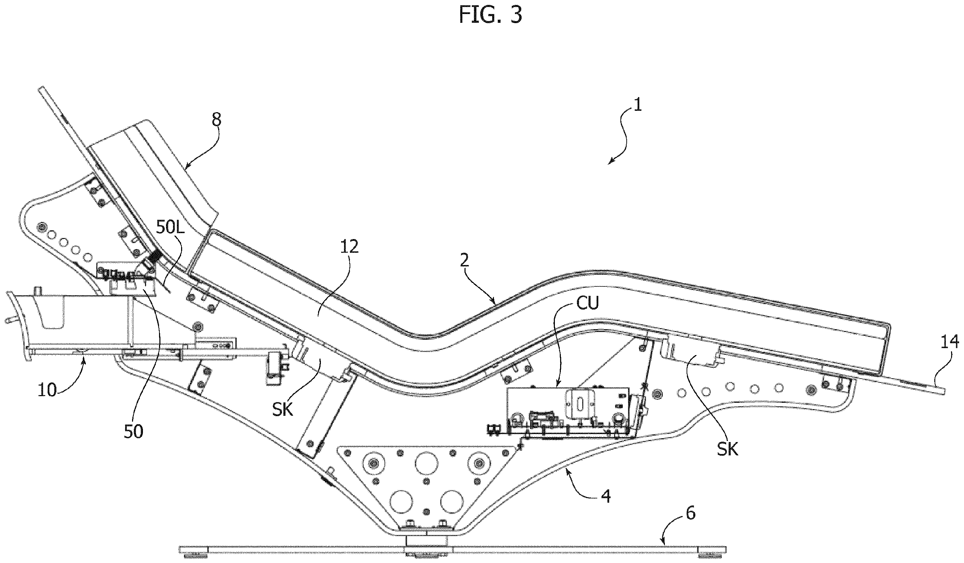

With reference to Figure 3, the hosting seat 2 is preferentially provided in the form of a mattress 12 that is mounted on an undulated plate 14, which is in turn fixed to the frame 4. The mattress 12 is preferentially made of expanded foam or shape-memory foam, and is always preferentially equipped with heating bands for regulating the temperature of the area for accommodating the user. Preferentially, the undulated plate 14 is moreover made of refractive material so as to be able to diffuse light stimuli coming from multicolour light sources provided therein.

The plate 14 is moreover shaped so as to provide the hosting seat 2 r with the outline of a reclined seat with raised legrest, typical of a chaise longue.

The frame 4 is configured as a hollow box-like body including a first substantially V-shaped side panel 16 and a second substantially V-shaped side panel 18, which are joined together by means of a fairing 20 that reproduces the peripheral profile of the panels 16, 18 in the areas not adjacent to the plate 14.

Within the frame 4 there finds place, among other things, the second stimulation unit 10 and an electronic control unit CU, which is operatively connected to all the stimulation units of the support 1 and is moreover operatively connected to a human-machine interface (or user interface) designated by the reference CI.

The interface CI may comprise a terminal with touchscreen or may be conceived as a station for connection to an electronic device owned by the user, such as a tablet or a smartphone on which a software application is installed provided by the manager of the support 1 (the software application may be provided also at the moment of the treatment by downloading it from a website administered by or on behalf of the manager of the support 1). By means of the interface CI the user interacts with the support 1 in the case of individual treatment, or receives predefined instructions in the case of group treatment according to modalities that will be described hereinafter.

With reference to Figures 4, 4A, and 5, there now follows a description of the first stimulation unit.

The first stimulation unit 8 is contained inside a cushion structure 22, which comprises a central portion 24, configured for receiving the user's head, and two wings 26 at opposite ends of the central portion - such as to be lateral with respect to the user's head - where the individual devices that administer the stimuli to the user are housed.

With reference to Figure 4A, the cushion structure 22 is provided by means of a plurality of cores (preferably, also these are made of expanded foam or shape-memory foam) assembled to define the final shape thereof and provided with an aesthetic covering. The cores comprise:
- a main core 28, in a central position, set on which is a plate 30, preferably made of soft and deformable material on which the head of the user rests; and
- two end cores that are substantially L-shaped (in cross section) and are designated by the reference numbers 32, 34; each end core 32, 34 is surmounted by an upper core 36, 38 projecting from the surface 24.

The main core 28 includes two plane and inclined lateral surfaces 40 located at opposite ends thereof, which accordingly face the cores 32, 34. In particular, the surfaces 40 face corresponding inclined plane surfaces 42, 44 of the end cores 32, 34 so as to define two acoustic horns 46, 48, here in the form of channels with rectangular cross section having a sectional area progressively decreasing from the inside towards the outside of the cushion structure 22.

Each acoustic horn 46, 48 gives out flush with the plate 30 and at the opposite end is closed by an acoustic diffuser or speaker SP.

According to an advantageous aspect of the invention, positioning of the speakers SP inside the respective acoustic horns 46, 48 is obtained by means of laminas L, which include a fastening flange L1, by means of which they are fixed to the base of the side cores 32, 34, a stem L2, which bears upon the core 28 immediately at the foot of the surface 40, a first inclined wing L3, positioned on which are the speakers SP and which defines with respect to the base L1 (i.e., to the base of the cushion structure 22) an angle of inclination α_{SP} preferentially chosen of 53° but in general comprised in the range 45°-60°. A second inclined surface L4 moreover bears upon the wall 42, 44, thus guaranteeing, together with the contact between the stem L2 and the sides of the core 28, correct positioning of the speakers SP inside the corresponding acoustic horn.

Each upper core 36, 38 is substantially wedge-shaped and incorporates a visual-stimulation device, which comprises one or more LED strips ST configured for diffusing visual stimuli through a screen SC.

Preferentially, the entire cushion structure 22 is coated with leather or eco-leather except for the area 24 that is coated with perforated leather so as not to hinder diffusion of sound through the acoustic horns 46, 48.

For aesthetic uniformity, the covering of the structure 22 is preferentially identical to that of the mattress 12.

With reference to Figures 6, 7, and 7A, and moreover with the aid of Figure 3, there now follows a description of the second stimulation unit 10.

The second stimulation unit 10 includes:
- a mobile element slidably mounted inside the frame between a retracted position and an extracted position and adapted to contain the consumable material; the mobile element is extractable to allow replacement of the depleted consumable material; and
- two (even though in general there may be one or more) stimulus-conveying elements installed in the above frame 4 and fixed with respect thereto (they constitute the fixed portion of the stimulation unit); the stimulus-conveying elements are configured for coupling to the mobile element when this is in the retracted position.

In this preferred embodiment, the fixed portion comprises, as stimulus-conveying elements, a first and a second hoods 50, each fixed to the frame 4 by means of a supporting lamina 50L (see Figure 3).

The mobile portion/element is defined, in the preferred embodiment illustrated herein, by a drawer 52, which is installed in the frame 4 and can be moved between a (closed) retracted position and an extracted position, visible in Figures 1 and 3, respectively. In the retracted position, the drawer 52 is preferentially of a hide-away type with respect to the shroud 20. As will be described more fully in what follows, the drawer/mobile element 52 can be pulled out to enable replacement of the depleted consumable material.

The drawer 52 is in particular slidably mounted inside the frame 4 between the retracted and extracted positions (for this purpose, rectilinear guides - here horizontal - are provided for movement of the drawer) and functions as housing for the consumable material of the stimulation unit 10 and for at least part of the stimulation devices that make up the unit 10.

The drawer 52 includes a central compartment 54, which is configured for housing accessories such as towels, cosmetics, or again devices for detection and monitoring of biological parameters of the user, such as a sensorized armband, or else a sensorized wristband (both sensitive to biological parameters, such as heart rate, skin conductance, cerebral activity), or else again a cardio-thoracic band.

The drawer 52 includes two lateral compartments 56, set within which is the consumable material with which the stimulation unit 10, configured for olfactory stimulation of the user/patient, operates.

In this embodiment, each compartment 56 includes three housings, housed inside which are corresponding vials or cartridges of essences F1, F2, F3 (it should be noted that their number may in general be any, typically from one to three/four for each compartment 56) .

The essence contained in each of the vials F1, F2, F3 is diffused via the device 10 in the direction of the user for stimulation of the olfactory apparatus. For this purpose, each hood 50 is fixed to the frame 4 - for example, by means of posts 60 with springs for recovery of play that engage the lamina 50L - in a position corresponding to the position occupied by a respective compartment 56 with respect to the frame 4 when the drawer 52 is in a closed position hidden away in the frame 4. In particular, the plan extension of the internal volume of each hood 50 is chosen so as to be equal to the plan extension of the compartment 56, so that each hood 50 can couple to the drawer 52 when it is in the retracted position, thus covering the corresponding compartment 56.

Each hood 50 includes a diffuser 58, a first end of which faces the compartment 56 and a second end of which gives out into the area 12 where the user is accommodated. In particular, the second end gives out into a specific portion of the area 12, i.e., in the area 24 of the cushion structure 22, through the perforated covering of the area 24 itself.

Set in a position substantially contiguous to each compartment 56 is a suction compartment 62 closed by a cover 64 that can be opened for inspection and maintenance. Set inside the compartment 62 is a battery of centrifugal blowers 66. The number of blowers 66 is equal to the number of housings into which the compartment 56 is divided, but in some variants it is possible to have a single centrifugal blower (in this case, the flow of air cannot be differentiated on the basis the essences installed).

The delivery of each centrifugal blower 66 is in fluid communication with a respective delivery duct 68, in particular an ascending duct (the delivery ducts are hence equal in number to the blowers), which communicates by means of an opening 70 with a corresponding housing of the compartment 56 where a phial F1, F2, F3 is housed.

The outlet of each duct 68 is in the internal volume of the hood 50 (which in the embodiment illustrated herein receives three outlets of ducts 68) in such a way that the fragrances of the phials F1, F2, F3 are mixed in the hood itself (entrainment thereof by the fluid current moved by the blower 66 is obtained by suction pressure through the opening 70) and exit therefrom in the form of a homogeneous suspension through the diffuser 58 for delivery to the user. Basically, when the drawer 52 is in a closed position, the hood 50 and the diffuser 58 constitute a continuation of the battery of ducts 68, connecting up to the latter and consequently establishing a fluid communication.

The treatment support 1 is completed by further stimulation units, which include:
- a visual stimulation unit comprising one or more perimetral strips PS of polychrome LEDs, which are embedded in grooves provided on the plate 14 (see Figure 2A) and diffuse the light through the material of the plate 14 itself; and
- a tactile-stimulation unit including one or more shakers SK (two in this embodiment) fixed to the plate 14 and in mechanical connection with the mattress 12.

Operation of the support 1 is described in what follows.

The support 1 can be used for administration of a variety of different treatments, in particular treatments that involve a generally multisensory stimulation. By means of the support 1 described herein it is possible to stimulate simultaneously or separately, according to the treatment protocol, hearing, sight, smell, and touch.

It will on the other hand be appreciated that the device 1 includes both stimulation units (the unit 8, but also the tactile-stimulation unit, or the further visual stimulation unit that uses the strips PS), operation of which is independent of supplies of consumable material, and a stimulation unit (the unit 10), operation of which depends upon supplies of consumable material.

The first stimulation unit 8 is configured for acoustic and visual stimulation by means of the speakers SP and the LED strips ST, respectively. Provision of the acoustic horns 46 and 48 enables the best possible conveyance of the sound towards the user's ears. It will be noted, in fact, that the positioning of the acoustic horns 46, 48, as well as that of the speakers SP therein (angle α_{SP}), are provided in such a way as to orient the acoustic stimuli in the direction of the corresponding receptor of the human body (outer ear and ear). Likewise the shape and inclination of the wings 26 (angle α26 equal to 20°, but in general ranging between 15° and 30°; the reference is always the bottom plane of the cushion structure 22 - or alternatively the plane of the plate 14 in the area of the structure 22) favours transmission of the stimulus to the user's eyes. Furthermore, preferentially the strips ST have a front surface inclined by 60° with respect to the bottom plane of the cushion structure 22 (angle α_{ST} in Figure 4A - for convenience, the reference is with respect to a plane parallel to the bottom plane).

Driving of the unit 8 is obtained, as mentioned previously, by means of the control unit CU. The user can adjust the volume of sound reproduction by means of the interface CI, and, in the case of individual treatment, may possibly select the type of light stimulus.

The olfactory apparatus of the user is, instead, stimulated by means of the stimulation unit 10. Via the control exerted by the control unit CU, it is possible to drive independently the rate of the electric motors that drive the individual centrifugal blowers 66 in order to regulate the amount of essence that, once released by the vial/cartridge F1, F2, F3 (by evaporation, sublimation, or similar mechanism), is entrained into each of the ducts 68 and expelled through the diffuser 58. The fragrance is diffused through the perforated coating of the area 24, where the diffusers 58 give out.

To provide a solution to the technical problem underlying the invention, the stimulation unit 10 that operates in a way depending upon supplies of consumable material includes a support such as the drawer 52 that can be pulled out to enable replacement of just the depleted consumable material or materials, without affecting operation of the other stimulation units, irrespective of the kind of consumable materials, and without requiring the support 1 to be dismantled, or much less without having to ask the user to descend temporarily from the support.

Disassenbly of the support 1 is not necessary thanks to the fact that the stimulus-conveying elements of the unit 10 - the hoods 50 - remain in a fixed position also when the drawer 52 is pulled out (i.e., during its movement towards the extracted position), which brings along with it only the compartments that house the consumable material and the batteries of centrifugal blowers 66. In this connection, the drawer 52 is equipped with a front switch SW that disconnects the supply of the blowers 66 as soon as the drawer 52 is opened.

In this way, in the case where the supplies of consumable material were to be depleted, the operator that assists the user during the treatment would simply have to pull out the drawer 52, take out the depleted consumable material (empty vials or depleted cartridges), and replace it with new material. The compartments 56, closed at the top by the hoods 50 when the drawer 52 is retracted inside the frame 4, are automatically exposed on the outside, thus also exposing the consumable material when the drawer 52 is pulled out, thus rendering replacement very convenient. It is to be noted that the replacement of the consumable material is possible both in the extracted end position (position of maximum extraction) and in the entire range of intermediate extracted positions where the compartments 56 are uncovered.

Preferentially, the drawer 52 is provided with an electric lock that can be unlocked only by authorised staff so as to limit unauthorized or accidental access (by patients, even though - as will be described shortly - the support 1 is conceived so as to be intrinsically secure in regard to any accidental opening).

Furthermore, extraction of the drawer 52 does not have any further impact on the administration of the treatment to the user in so far as administration is made in a way completely transparent to the user. In fact, arrest of the blowers 66 does not entail the arrest of any other stimulation device present on board the support 1, and likewise does not lead to any interference with the user who is resting in the area provided for accommodating him or her since the removable support (drawer 52) is set in a position shielded with respect to the seat itself.

Here in particular, it will be noted, the drawer 52 is installed in the frame 4 on a side opposite to the seat 2, in particular on an opposite side of the plate 14, thus rendering the drawer 52 physically unreachable by the user who is undergoing treatment. In this way, the support 1 is intrinsically secure in regard to any accidental access to the drawer 52.

The time of replacement of the consumable material is in practice so short that the user cannot perceive any intervention by the operator: the short interruption in the delivery of the olfactory stimuli is in any case compensated by the presence of air already diffused and mixed with fragrances as a consequence of the previous delivery so that any discomfort for the user is minimal, if not altogether absent.

Finally, it should be noted that the support 1 lends itself to some modifications that give rise to alternative embodiments.

For instance, it is possible to envisage embodiments in which also the stimulus-conveying elements are fixed with respect to the mobile element 52. In these embodiments, the mobile element 52 is provided as a drawer that occupies a portion of the shroud 20, which extends at least for an area corresponding to the projection on the shroud 20 of the cushion structure 22. In this way, the devices of the unit 10 can be positioned and sized so that the hood or hoods 50 with the corresponding diffusers can be positioned automatically behind the area 24 when the drawer 52 is brought into the retracted position. When, instead, the drawer 52 is in an extracted position, the hood 50 or hoods 50 may be temporarily removed by the operator, for example, by disengaging slotted coupling elements.

For instance, it is possible to envisage a single hood 50 set in a position corresponding to the compartment 54 when the drawer 52 is retracted inside the frame 4. In this case, the compartment 54 would function as container for the consumable material and would be associated, just as in the case of the compartments 56 in the preferred embodiment described, to one or more - preferably an array of - centrifugal blowers and corresponding ascending ducts 68 for connection to the hood 50. The diffuser 58 would in this case be just one, but it is also possible to envisage that the same embodiment is equipped with a diffuser 58 with dual outlet (with Y-shaped path) for conveying the olfactory stimulus into two areas close to the surface 24 exactly as occurs in the preferred embodiment.

Furthermore, there may be envisaged embodiments in which only the stimulation unit 10 is present, i.e., the one that depends upon supplies of consumable materials, in particular for olfactory stimulation. The unit 10 may again be provided on the basis of any one of the possibilities already described: with double hood 50 or single hood 50 (both with hood or hoods 50 fixed to the frame 4), or else with (single or double) hood that can be extracted along with the drawer 52. Whatever the solution chosen, in these embodiments, the cushion-like structure 22 would constitute a simple headrest, and would be provided as a normal padded cushion, possibly provided with wings similar to the wings 26 but with the sole function of lateral containment of the user's head.

In addition, in the embodiments in which the stimulation unit 10 is the only unit present, there may be envisaged the possibility of retrofitting of the stimulation unit 8, which can be supplied as accessory that substitutes the headrest and is connected to the control unit CU by means of a so-called plug-and-play logic. The same applies to the remaining stimulation units described, operation of which is independent of consumable materials, which also may be associated to a possibility of retrofit. For instance, these stimulation units can be provided again as separate accessories that can be installed on the support 1 subsequently and can be connected to the control unit CU by means of a plug-and-play logic. The control unit CU may be originally programmed by means of a software that enables management of all the possible stimulation units and that is executed limitedly to the portions that correspond to the devices that are connected up, or else may be provided with a software that can be updated at the moment of retrofitting with the further stimulation modules referred to above.

By means of a number of supports 1 according to the invention it is moreover possible to define either a complex of stations for individual treatment or a kit for group treatment of patients. The support 1 is in fact equipped with Ethernet, USB, HDMI interfaces and is moreover provided with Bluetooth® and Wi-Fi connectivity that enables the widest possible freedom of management.

Described hereinafter in detail are two methods of delivery of treatments to a user that differ according to the use of the supports 1.

Individual treatment mode ("host"): the end user, through the interface CI, can select the most suitable course of treatment and manage the different stimulations (regulation of the volume, pause or arrest of the treatment protocol, activation and/or deactivation of stimulations). The functions available for the host user are in any case limited: for example, the user will not be able to alter the treatment protocol.

Group-treatment mode ("operator"): the manager of the treatment centre by means of an external terminal (e.g., a tablet) connected via Bluetooth® or Wi-Fi to the various supports 1 will be able to select for the treatment just one support 1 or a number of supports 1, and possibly form groups of supports 1 with treatment courses different for each group, or else one and the same course for all the groups. The manager can direct and manage simultaneously the protocols on the basis of his activities. The functions available to the operator are not limited: he can intervene during the protocol on the stimulations, co-ordinating different activities. Basically, this is a control logic with star hierarchy in which the external treatment under the control of the operator operates with a "master" function, whereas the individual supports 1 operate (and are consequently controlled) as "slave" devices.

Of course, the details of construction and the embodiments may vary widely with respect to what is described and illustrated herein, without thereby departing from the scope of the present invention, as defined by the annexed claims.

## Claims

1. A support (1) for hosting of a treated subject, particularly a chaise longue, including a hosting seat (2) configured to host a user, and a frame (4) carrying said hosting seat (2),
the support (1) comprising a stimulation unit (10) including one or more stimulation devices (66, 50) configured for operating dependently of consumable material (F1, F2, F3),
wherein said stimulation unit (10) includes a mobile element (52) installed within said frame (4) and movable between a retracted position and an extracted position, and configured to contain the consumable material (F1, F2, F3), said mobile element (52) being extractable to allow the replacement of the depleted consumable material
the support (1) being **characterized in that** it includes at least one further stimulation unit (8) including one or more stimulation devices (SP, ST) configured for operating independently of consumable material,
said further stimulation unit (8) includes a cushion structure (22) provided with two wings (26) at opposite ends thereof,
wherein said cushion-like structure (22) is positioned at an end of the hosting seat (12) configured to receive the head of a user, and
wherein in correspondence of each of said wings (26) a device for visual stimulation (ST, SC) and/or a device for sound stimulation (SP) are arranged,
wherein said cushion structure (22) houses a first and a second speaker (SP) in correspondence of each of said wings (26), each speaker (SP) being installed in a corresponding acoustic horn (46, 48).

2. The support (1) according to Claim 1, wherein said stimulation unit (10) includes one or more stimulus conveying elements (50) installed within said frame (4) and fixed with respect thereto, said stimulus conveying elements (50) being configured for coupling with said mobile element (52) when the mobile element (52) is in said retracted position.

3. The support (1) according to any of claims 1 or 2, wherein said second stimulation unit (10) is configured for olfactory stimulation,
wherein said mobile element (52) is a drawer including a first and a second side compartments (56) adapted to contain the consumable material (F1, F2, F3) and
wherein furthermore said stimulation unit (10) includes a first and a second stimulus conveying elements (50) configured to cover a corresponding one of said side compartments (56) when the drawer (52) is in the retracted position within the frame (4).

4. The support (1) according to claim 3, wherein each stimulus conveying element (50) is a hood (50) including a diffuser (58) that gives out in correspondence of said hosting area (12, 8; 24), said hood (50) having a plan extension equal to the plan extension of corresponding one of said lateral compartments (56).

5. The support (1) according to claim 4, wherein one or more centrifugal blowers (66) are associated to each of the side compartments (56), each of the centrifugal blowers being in communication with a corresponding ascending conduit (68) configured for joining with said diffuser (58) when the drawer (52) is in the retracted position, said ascending conduit (68) being provided with an aperture (70) through which the consumable material (F1, F2, F3) in the side compartment (56) is entrained into the fluid stream processed by the corresponding centrifugal blower (66) during the operation of the stimulation unit (10).

6. The support (1) according to claim 5, wherein each of the side compartments (56) includes a plurality of housings, each containing a consumable material cartridge (F1, F2, F3) and wherein the stimulation unit (10) includes a stack of centrifugal blowers (66) in equal number to the number of housings of the corresponding side compartment (56), and ascending conduits (68) in number equal to the number of centrifugal blower (66), wherein each ascending conduit (68) joints with said diffuser (58) when the drawer (52) is in the retracted position into the frame (4).

7. The support (1) according to claim 1, wherein said cushion structure (22) includes a main core (28) and two side cores (32, 34), said main core (28) including a first and a second inclined lateral surfaces facing towards a corresponding one of said wings, and each of said side cores (32, 34) including a respective inclined surface (42, 44) facing a corresponding inclined lateral surface (40) of said main core (28), defining thereby a corresponding one of said acoustic horns (46, 48) when the cores are assembled to form the cushion structure (22).

## Patentansprüche

1. Unterlage (1) zum Tragen eines behandelten Individuums, insbesondere Liegestuhl, die einen Tragsitz (2), der dafür ausgelegt ist, einen Nutzer zu tragen, und einen den Tragsitz (2) tragenden Rahmen (4) beinhaltet, wobei die Unterlage (1) eine Stimulationseinheit (10) umfasst, die eine oder mehrere Stimulationseinrichtungen (66, 50) beinhaltet, die dafür ausgelegt sind, abhängig von Verbrauchsmaterial (F1, F2, F3) zu arbeiten,
wobei die Stimulationseinheit (10) ein mobiles Element (52) beinhaltet, das in den Rahmen (4) eingebaut und zwischen einer eingefahrenen Position und einer ausgefahrenen Position bewegbar ist und dafür ausgelegt ist, das Verbrauchsmaterial (F1, F2, F3) zu enthalten, wobei das mobile Element (52) ausfahrbar ist, um das Ersetzen des aufgebrauchten Verbrauchsmaterials zu ermöglichen,
wobei die Unterlage (1) **dadurch gekennzeichnet ist, dass** sie mindestens eine weitere Stimulationseinheit (8) beinhaltet, die eine oder mehrere Stimulationseinrichtungen (SP, ST) beinhaltet, die dafür ausgelegt sind, unabhängig von Verbrauchsmaterial zu arbeiten,
die weitere Stimulationseinheit (8) eine Kissenstruktur (22) beinhaltet, die an ihren entgegengesetzten Enden mit zwei Flügeln (26) versehen ist, wobei die kissenartige Struktur (22) an einem zum Aufnehmen des Kopfes eines Nutzers ausgelegten Ende des Tragsitzes (12) positioniert ist und
wobei in Entsprechung zu jedem der Flügel (26) eine Einrichtung für optische Stimulation (ST, SC) und/oder eine Einrichtung für Schallstimulation (SP) angeordnet sind,
wobei die Kissenstruktur (22) einen ersten und einen zweiten Lautsprecher (SP) in Entsprechung zu jedem der Flügel (26) enthält, wobei jeder Lautsprecher (SP) in einen entsprechenden Schalltrichter (46, 48) eingebaut ist.

2. Unterlage (1) nach Anspruch 1, wobei die Stimulationseinheit (10) ein oder mehrere Reizübertragungselemente (50) beinhaltet, die in den Rahmen (4) eingebaut und in Bezug auf diesen fixiert sind, wobei die Reizübertragungselemente (50) für eine Kopplung mit dem mobilen Element (52) ausgelegt sind, wenn sich das mobile Element (52) in der eingefahrenen Position befindet.

3. Unterlage (1) nach einem der Ansprüche 1 oder 2, wobei die zweite Stimulationseinheit (10) für Geruchsstimulation ausgelegt ist,
wobei es sich bei dem mobilen Element (52) um eine Schublade handelt, die ein erstes und ein zweites Seitenfach (56) beinhaltet, die dafür ausgelegt sind, das Verbrauchsmaterial (F1, F2, F3) zu enthalten, und wobei ferner die Stimulationseinheit (10) ein erstes und ein zweites Reizübertragungselement (50) beinhaltet, die dafür ausgelegt sind, ein entsprechendes der Seitenfächer (56) abzudecken, wenn sich die Schublade (52) in der eingefahrenen Position in dem Rahmen (4) befindet.

4. Unterlage (1) nach Anspruch 3, wobei es sich bei jedem Reizübertragungselement (50) um eine einen Diffusor (58) beinhaltende Haube (50) handelt, die in Entsprechung zu dem Tragbereich (12, 8; 24) ausgibt, wobei die Haube (50) eine ebene Ausdehnung aufweist, die der ebenen Ausdehnung eines entsprechenden der Seitenfächer (56) gleicht.

5. Unterlage (1) nach Anspruch 4, wobei jedem der Seitenfächer (56) ein oder mehrere Zentrifugalgebläse (66) zugeordnet sind, wobei jedes der Zentrifugalgebläse mit einem entsprechenden Steigkanal (68) in Verbindung steht, der dafür ausgelegt ist, sich mit dem Diffusor (58) zu verbinden, wenn sich die Schublade (52) in der eingefahrenen Position befindet, wobei der Steigkanal (68) mit einer Öffnung (70) versehen ist, durch die das Verbrauchsmaterial (F1, F2, F3) in dem Seitenfach (56) während des Betriebs der Stimulationseinheit (10) in den durch das entsprechende Zentrifugalgebläse (66) entwickelten Fluidstrom zugegeben wird.

6. Unterlage (1) nach Anspruch 5, wobei jedes der Seitenfächer (56) eine Vielzahl von Gehäusen beinhaltet, die jeweils eine Verbrauchsmaterialkartusche (F1, F2, F3) enthalten, und wobei die Stimulationseinheit (10) eine Stapelung aus Zentrifugalgebläsen (66), deren Anzahl der Anzahl der Gehäuse des entsprechenden Seitenfachs (56) gleicht, und Steigkanälen (68) beinhaltet, deren Anzahl der Anzahl der Zentrifugalgebläse (66) gleicht, wobei sich jeder Steigschacht (68) mit dem Diffusor (58) verbindet, wenn sich die Schublade (52) in der in den Rahmen (4) eingefahrenen Position befindet.

7. Unterlage (1) nach Anspruch 1, wobei die Kissenstruktur (22) einen Hauptkern (28) und zwei Seitenkerne (32, 34) beinhaltet, wobei der Hauptkern (28) eine erste und eine zweite geneigte Seitenfläche beinhaltet, die einem entsprechenden der Flügel zugewandt sind, und wobei jeder der Seitenkerne (32, 34) eine jeweilige geneigte Fläche (42, 44) beinhaltet, die einer entsprechenden geneigten Seitenfläche (40) des Hauptkerns (28) zugewandt ist, wodurch diese einen entsprechenden der Schalltrichter (46, 48) definieren, wenn die Kerne zum Bilden der Kissenstruktur (22) zusammengesetzt sind.

## Revendications

1. Support (1) pour l'accueil d'un sujet traité, en particulier une chaise longue, comportant un siège d'accueil (2) configuré pour accueillir un utilisateur, et un cadre (4) portant ledit siège d'accueil (2),
le support (1) comprenant une unité de stimulation (10) comportant un ou plusieurs dispositif(s) de stimulation (66, 50) configuré(s) pour fonctionner de manière dépendante du matériau consommable (F1, F2, F3),
dans lequel ladite unité de stimulation (10) comporte un élément mobile (52) installé dans ledit cadre (4) pouvant être déplacé entre une position rétractée et une position déployée, et configuré pour contenir le matériau consommable (F1, F2, F3), ledit élément mobile (52) étant extractible pour permettre le remplacement du matériau consommable appauvri,
le support (1) étant **caractérisé en ce qu'**il comporte au moins une unité de stimulation supplémentaire (8) comportant un ou plusieurs dispositif(s) de stimulation (SP, ST) configuré(s) pour fonctionner indépendamment du matériau consommable, ladite unité de stimulation supplémentaire (8) comporte une structure de coussin (22) munie de deux ailes (26) à ses extrémités opposées,
dans lequel ladite structure en forme de coussin (22) est positionnée à une extrémité du siège d'accueil (12) configurée pour recevoir la tête d'un utilisateur, et
dans lequel, en correspondance de chacune desdites ailes (26), un dispositif de stimulation visuelle (ST, SC) et/ou un dispositif de stimulation sonore (SP) est/sont agencé(s),
dans lequel ladite structure de coussin (22) accueille des premier et deuxième haut-parleurs (SP) en correspondance de chacune desdites ailes (26), chaque haut-parleur (SP) étant installé dans un pavillon acoustique (46, 48) correspondant.

2. Support (1) selon la revendication 1, dans lequel ladite unité de stimulation (10) comporte un ou plusieurs élément(s) de transport de stimulus (50) installé(s) dans ledit cadre (4) et fixé(s) par rapport à celui-ci, lesdits éléments de transport de stimulus (50) étant configurés pour se coupler audit élément mobile (52) lorsque l'élément mobile (52) se trouve dans ladite position rétractée.

3. Support (1) selon l'une des revendications 1 et 2, dans lequel ladite deuxième unité de stimulation (10) est configurée pour une stimulation olfactive,
dans lequel ledit élément mobile (52) est un tiroir comportant des premier et deuxième compartiments latéraux (56) adaptés pour contenir le matériau consommable (F1, F2, F3), et
dans lequel, en outre, ladite unité de stimulation (10) comporte des premier et deuxième éléments de transport de stimulus (50) configurés pour recouvrir un compartiment correspondant desdits compartiments latéraux (56) lorsque le tiroir (52) se trouve dans la position rétractée dans le cadre (4).

4. Support (1) selon la revendication 3, dans lequel chaque élément de transport de stimulus (50) est une hotte (50) comportant un diffuseur (58) qui diffuse en correspondance de ladite zone d'accueil (12, 8 ; 24), ladite hotte (50) ayant une extension en plan égale à l'extension en plan d'un compartiment correspondant desdits compartiments latéraux (56).

5. Support (1) selon la revendication 4, dans lequel un ou plusieurs ventilateur(s) centrifuge(s) (66) est/sont associé(s) à chacun des compartiments latéraux (56), chacun des ventilateurs centrifuges étant en communication avec un conduit ascendant correspondant (68) configuré pour être relié audit diffuseur (58) lorsque le tiroir (52) se trouve dans la position rétractée, ledit conduit ascendant (68) étant muni d'une ouverture (70) à travers laquelle le matériau consommable (F1, F2, F3) dans le compartiment latéral (56) est entraîné dans le courant de fluide traité par le ventilateur centrifuge correspondant (66) pendant le fonctionnement de l'unité de stimulation (10).

6. Support (1) selon la revendication 5, dans lequel chacun des compartiments latéraux (56) comporte une pluralité de boîtiers contenant chacun une cartouche de matériau consommable (F1, F2, F3) et dans lequel l'unité de stimulation (10) comporte un empilement de ventilateurs centrifuges (66) en nombre égal au nombre de boîtiers du compartiment latéral correspondant (56), et des conduits ascendants (68) en nombre égal au nombre de ventilateurs centrifuges (66), où chaque conduit ascendant (68) est relié audit diffuseur (58) lorsque le tiroir (52) se trouve dans la position rétractée dans le cadre (4).

7. Support (1) selon la revendication 1, dans lequel ladite structure de coussin (22) comporte un noyau principal (28) et deux noyaux latéraux (32, 34), ledit noyau principal (28) comportant des première et deuxième surfaces latérales inclinées faisant face à une aile correspondante desdites ailes, et chacun desdits noyaux latéraux (32, 34) comportant une surface inclinée respective (42, 44) faisant face à une surface latérale inclinée correspondante (40) dudit noyau principal (28), définissant ainsi un pavillon correspondant desdits pavillons acoustiques (46, 48) lorsque les noyaux sont assemblés pour former la structure de coussin (22).
